# EUROPEAN PATENT SPECIFICATION

(11) **EP 4 598 340 B1**
(45) Date of publication and mention of the grant of the patent: **05.08.2026**
(21) Application number: 23785884.0
(22) Date of filing: 03.10.2023
(51) Int. Cl.: G01N 21/84, A01J 5/013, G01N 21/78, G01N 33/04

(54) **MILKING SYSTEM WITH ANALYSIS UNIT**
MELKSYSTEM MIT ANALYSEEINHEIT
SYSTÈME DE TRAITE AVEC UNITÉ D'ANALYSE

(30) Priority: 06.10.2022 NL 2033247
(43) Date of publication of application: 13.08.2025
(73) Proprietor: Lely Patent N.V., 3147 PB Maassluis (NL)
(72) Inventor: MCCHESNEY, Darby Anne, 3147 PB Maassluis (NL); PULHAC, Peter James, 3147 PB Maassluis (NL)
(74) Representative: Octrooibureau Van der Lely N.V.
(86) International application number: PCT/IB2023/059891
(87) International publication number: WO 2024/074991

(56) References cited:
- WO-A1-2020/067882

## Description

The present invention relates to a milking system for sampling and analysing milk from a dairy animal.

In the dairy industry it is very important to have a thorough knowledge of the health of cows, as well as of the composition of the milk. This determines to a certain extent which health measures to take and/or what feed composition is best suited for the animals. Milk of cows is regularly sampled and sent to a laboratory for analysis. However, the results are not only available only (many) days later, which is of little use for day-today management, but it is also a relatively labour intensive and costly measure, which is undesirable.

To counter these disadvantages, in particular but not exclusively in the realm of robotic milking, in which dairy animals are milked without human supervision, automation of the sampling and analysis is desirable. In the state of the art, there is known for example WO2007/082545, which discloses a dry stick device for sampling milk. The dry stick comprises optionally a solid support, a reagent pad with a reagent, and a development pad with a controlling compound for providing a reaction condition.

Although the known dry stick device is in itself suitable for determining a compound in a milk sample, there is no disclosure of how to use the dry stick device in an automated system suitable for robotic milking. Such a system should be able to keep a plurality of reagent pads, for a similar plurality of sampling moments, available in an environment that is suitable for long-time storage, and to provide a new reagent pad in an easily controllable way. Thereto, reference is made to e.g. WO2020067882 and WO2020/067883, which disclose a milking system in which a milk sample is taken from a milk line, and a droplet thereof is provided to a reagent pad that is present on a carrier. The carrier is movable, such that for a new sampling a new reagent pad is available. However, the latter documents do not disclose anything about a system for a certain type of analysis tape with a priming agent, that is suitable for working for a long time in a dairy industry environment without special measures, in particular with a vertical flow reagent pad that is chemically stable for a long time, and that does not require special handling measures to ensure reliable results.

The present invention aims to provide such a milking system according to claim 1.

According to the present invention, the milking system comprises a cassette with an analysis tape that carries the analysis pads. A tape as carrier is easily manipulated, as is known according to e.g. WO2020067882 and WO2020/067883, and also for example in tape recorders. The analysis pads may be safely kept in a storage cassette or the like. The analysis pads each comprise a reagent pad and a priming pad, which should at the same time be bonded together, kept apart chemically and allow sample (milk) fluid to go from the priming pad to the reagent pad, or vice versa. This is achieved by providing an adhesive layer between the two pad types. In order for fluid to be able to pass between the two pad types, there are holes in the adhesive layer. Preferably, these holes have been provided before applying the adhesive layer to one of the other parts of the analysis pads or carrier tape. Furthermore, the adhesive layer bonds together two layers, and therefore, for easy handling, requires on both sides thereof a protective liner that is peeled off before bonding to a next layer. The process of making holes in the adhesive layer runs the risk of inadvertently joining together the adhesive layer and the liner too tightly, which in turn leads to the risk of the adhesive layer sticking to the liner being peeled off before use, instead of to the other liner on the other side of the adhesive layer. To counter this, according to the present invention, the adhesive layer is provided as a "bonding layer", which is a combination of a first adhesive layer, a support layer, and a second adhesive layer. The holes are made in the bonding layer as a whole, then still including the liners which are present on both sides of the adhesive layer before applying the bonding layer to the reagent pad or priming pad. Removing the first of the two liners enables bonding to one of the reagent pad or priming pad. While removing the first liner, dimensional stability is provided by the support layer. Furthermore, the problem of the liner sticking to the (first or second) adhesive layer can be minimised by providing a (much) stronger bond between the respective adhesive layer and the support layer. This is easy to do because of the inherent properties of the liner, the adhesive layer and the support layer. After applying the bonding layer to the first of the pad types, the second liner is peeled off, and the second pad type is applied to the other adhesive layer.

The milking system will thus provide reliable measuring results during a long time without human interference, because the analysis pads show a good separation of the reagent pad and the priming pad, thus ensuring a weak to absent chemical reaction between the chemicals in the two pad types. Thus, a cassette with a large number of analysis pads may be stored in the milking system for a long time, even without cooling or the like, as long as ingress of dust and humidity is minimised. The latter is relatively easy, even in a dirty dairy environment. Furthermore the holes between the two pad types that allow fluid (milk) to migrate from one pad type to the other will be dimensionally stable, and will also still be provided in the original pattern. The reagent pads will therefore provide a good (optical) response without irregularities due to an undesired change of the distribution of the holes. A further advantage is that the support layer provides additional strength to the analysis pads. Thus, when the tape mover moves the analysis tape to bring a new analysis pad in a position for receiving a sample droplet, there will be even less deformation that might affect the measurement reliability.

It was found by the inventors that a pore diameter of below 0.15 mm did not provide satisfactory results, while a pore size between about 0.15 and about 0.18 mm provided at least somewhat satisfactory results, as to definition and regularity of the colour image. From a pore diameter of 0.18 mm, the overall results are good. However, it is to be noted that these numbers could be influenced by the type of milk to be tested. Without wishing to be tied to an explanation, it is suggested that fat globules present in whole milk may have some influence on the behaviour of the milk in the analysis pad. If so, it could be that for skimmed, fat-free milk, the minimum pore size could be smaller. However, for fresh, whole non-homogenised milk (at least from cows) the indicated numbers are believed to represent limits of a non-workable range, a more or less satisfactory range, and a good range.

Additional embodiments, features and advantages will become apparent upon reading the attached claims as well as the below detailed description of the invention.

In some embodiments, said plurality of holes is arranged in a regular pattern. This holds in particular for the centres of the holes. Although it is noted that it is not necessary to provide the holes in a regular pattern, the more regular the pattern, the more evenly distributed the milk, and thus the colour reaction, will be in the pad. Thus, any detection of the colour detection, be it performed by a human, or automatically by e.g. a colorimetric arrangement, may become more reliable. The pattern is in particular a square pattern, which is a simple pattern that is easily made to be regular, and ensures symmetry and regularity in many directions. Yet other patterns are not excluded, such as lozenge-shaped patterns, rectangular patterns, and so on.

The holes may have been provided by any known method or apparatus, such as by etching or mechanical means, which may lead to irregular hole distribution. A useful method or means to provide the holes is by means of a laser tool. This allows a very good control over position and size of the holes. This will be further elucidated in relation to the third aspect of the present invention.

In embodiments, said pattern has a hole centre to hole centre spacing of 1.5 times and 2.5 times said diameter of the holes. This provides a good balance between mechanical strength of the tape and good flow-through properties of the sample from layer to layer.

In embodiments, the carrier tape has a longitudinal direction, and said pattern extends mirror symmetrically with respect to said longitudinal direction. Because in most cases the pads will be provided lengthwise on the tape, a pattern with a lengthwise mirror symmetry allows a simple way of ensuring that all pads have more or less the same distribution of the holes over its surface area. In particular for smaller pads, with a relatively lower number of holes, this allows a better comparison from pad to pad, and thus a higher reliability and mutual comparison when assessing any colour reaction in the pads. Furthermore, it also provides a useful direction when preparing the holes in the adhesive layer.

In embodiments, the analysis pad has a maximum dimension, in particular a length, and the diameter of the holes is at most 1/10th of said maximum dimension, more in particular at most 1/20th of said maximum dimension. Although larger holes could allow a higher flow rate of fluid (milk) through the analysis tape during use thereof, the inventors have found that the indicated relative hole size gives a better definition of the colour reaction, which increases the reliability of the measurements.

In embodiments, the bonding layer has a thickness in the range of 0.02-0.01 mm, preferably a thickness of 40 ± 20 µm, i.e. a thickness in the range 20-60 µm, such as about 1 mil (=1/1000th of an inch). With such layer thickness, the desired properties of strength, separation of the layers with the agent(s), sufficient cross-over between the bonded layers that allows a good flow rate of fluid through the tape, et cetera, were found to be optimum for milk. Yet, for other circumstances and/or sample materials, such as possibly for skimmed milk or the like, a different layer thickness could prove even better.

In advantageous embodiments, the compound is milk urea nitrogen (MUN). This is an important component in milk, providing e.g. an indication about the feeding and health of the animal. But in many cases, if not all useful cases, the reagent that is to give a colour reaction with the milk urea nitrogen from the milk is a component that requires specific conditions to give optimum results while having a suboptimal chemical stability under those conditions. In other words, many, if not all, useful agents for providing a colour reaction with milk urea nitrogen need some form of priming. A vertical flow set-up for the analysis tape is then a very suitable set-up, that provides a relatively high flow rate through the pad, yet also a long-term stability because of the possibility to separate the agent from the priming agent. Of course it is possible to use other, targeted reagents for one or more other compounds in milk. Examples are lactate-dehydrogenase (LDH), beta-hydroxybutyrate (BHB), milk fat (triglycerides) and fatty acids (e.g. non-esterified fatty acids or NEFA), somatic cell count (SCC), total protein, and sugars such as glucose and lactose.

In embodiments, said priming agent comprises an acid, preferably providing a pH ≤ 2. Of course, such pH value will only be achieved after dissolution of the acid in the milk flowing through the reagent pad, i.e. after provision of the sample. Examples of useful acids are fumaric acid, phthalic acid, sulfamic acid and sulfonic acid. For other compounds to be detected, or for a different purpose, a different or additional priming agent may be desirable, such as an alkali to provide a high pH value, an enzyme or the like to clarify the (milk) sample, and so on.

The first and second adhesive layers may be selected according to the materials they need to bond to, and may differ from each other. Useful examples of adhesives are acrylic adhesives, silicone adhesives and (natural) rubber adhesives. The support layer between the adhesive layers, as well as the carrier layer of the tape, may be selected according to e.g. the type(s) of adhesive and/or priming agent or reagent chosen, and may be a polymeric material such as PET or polyester. The polymeric material, in particular that of the carrier layer of the tape, is preferably transparent and colourless.

In a second aspect, the invention relates according to claim 9 to a vertical flow analysis tape for milk analysis, arranged to be provided in the milking system according to the first aspect of the present invention. The analysis tape according to this second aspect comprises a carrier layer with a plurality of separate vertical flow analysis pads provided thereon, wherein each analysis pad comprises a reagent pad, comprising a liquid-permeable material and a reagent that is arranged to react with a predetermined compound in the sample to give a response that is detectable by the analysis system, a priming pad, comprising a liquid-permeable material and a priming agent that is arranged to change a property of the sample that enhances the response, and a bonding layer between the reagent pad and the priming pad, the bonding layer comprising a first adhesive layer, a second adhesive layer, and a support layer between said two adhesive layers, wherein the bonding layer has a plurality of throughgoing holes, each hole having a diameter of at least about 0.15 mm. The advantages of such a vertical flow analysis tape are in principle the same as mentioned for the first aspect of the present invention, and need not be repeated here. However, it is stressed that these are very prominent when such a tape is used in a robotic milking arrangement, in which the tape may be used reliably for a relatively long time. It is furthermore noted that the advantages as mentioned for the particular embodiments of the milking system according to the first aspect also hold for the vertical flow analysis tape according to this second aspect, unless the context teaches otherwise.

The liquid permeable material of the pads may comprise many types of material, such as cellulose, glass fibres or fibres of polyester, nylon, poly(ether)sulfone, polypropylene, and so on. It is also possible to use different types of material for different layers, such as a first liquid permeable material for the priming agent layer, and a different liquid permeable layer for the reagent, in each case of course optimised for the specific priming agent or reagent used.

In a third aspect, the invention relates according to claim 10 to a method for producing the vertical flow analysis tape of the second aspect of the present invention. The method comprises the steps of providing a bonding layer that comprises a first adhesive layer, a second adhesive layer, and a carrier layer between said two adhesive layers, making a pattern of throughgoing holes in the bonding layer, preferably by means of a laser tool, wherein the holes have a diameter of at least about 0,15 mm, providing a layer of reagent pad material, comprising a liquid-permeable material and a reagent that is arranged to react with a predetermined compound when supplied to the reagent pad material to give a response that is detectable by an analysis system, and providing a layer of priming pad material, comprising a liquid-permeable material and a priming agent that is arranged to change a property of the sample that enhances the response, wherein one of the reagent pad material and the priming pad material is supplied on a carrier layer, bonding the bonding layer to the reagent pad material and to the priming pad material, and separating the bonded reagent pad material, bonding layer and priming pad material into separate analysis pads, preferably by means of laser cutting. Obviously, the reagent will react with the compound after supply of the sample. The method steps need not all be performed in the indicated order, although some will necessarily follow one or more other steps. This method is eminently suitable for producing the vertical flow analysis tape of the second aspect. It provides the tape with very predictable flow properties, and thus with high reliability when in use.

The invention will now be elucidated further with reference to the drawings, showing a number of non-limiting embodiments, and in which
- Figure 1 very diagrammatically shows a milking system according to the invention;
- Figure 2 diagrammatically shows some details of the analysis device 5 of the milking system 1 according to the invention;
- Figure 3 diagrammatically shows a cross-sectional view through an analysis tape 10 according to the invention;
- Figure 4 diagrammatically shows a top view of an analysis tape 10 according to the present invention, and
- Fig. 5 shows a possible first step in a method of producing of the analysis tape according to the invention.

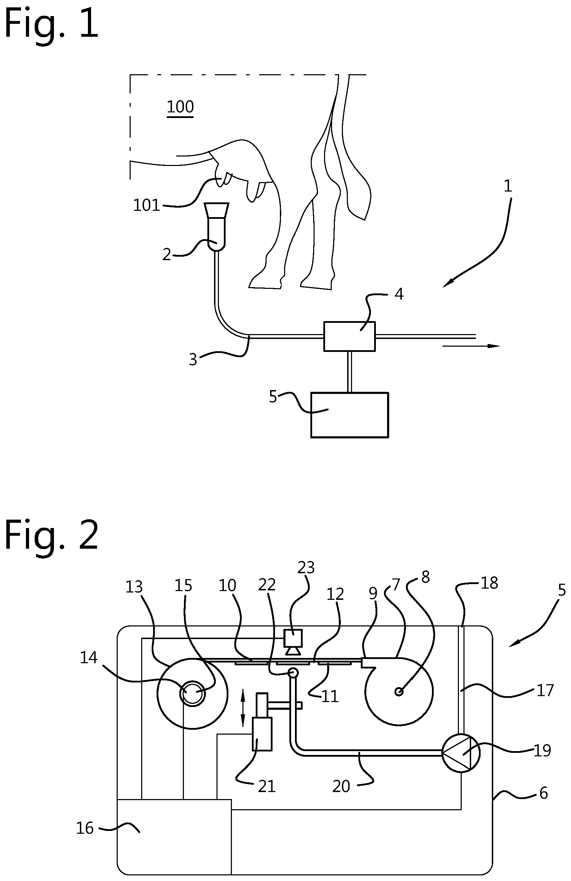

Figure 1 very diagrammatically shows a milking system according to the invention. The milking system is indicated with reference numeral 1, and comprises a teat cup 2 connected to a milk tube 3, from which a sampling device 4 extracts a milk sample that is sent to the analysis device 5. A cow 100 has teats 101.

In practice, the milking system 1 will comprise many more parts that are not shown here for clarity and conciseness. Examples are a vacuum system for providing a milking vacuum and a pulsation vacuum, optionally a milking box, and so on. Furthermore, although only one teat cup 2 is shown, the actual number of teat cups will of course be adapted to the animal to be milked. For example goat milking systems will have two teat cups, while cow milking systems will have four.

In use of the milking system 1, milk will be drawn from the teat 101 by the teat cup 2. This milk will flow through the milk tube 3 towards either a milk jar or directly to a milk tank, neither being shown but indicated by the arrow. The sampling device 4 will produce a sample from the milk and sends this to the analysis device 5. Herein, the milk tube 3 may be a so-called short milk tube, that connects the teat cup 2 to either a milk jar (in a robotic milking system) or to a milk claw (in a conventional milking system). An advantage of sampling from the short milk tube is that milk from an individual teat 101 may be analysed. This is advantageous for compounds that may differ in concentration from teat tot teat, such as somatic cells. However, in turn this requires at least a corresponding number of sampling devices, and possibly also of analysis devices, which adds complexity to the system. Furthermore, many milk compounds are distributed evenly over the parts (often "quarters") of a dairy animal's udder, such as most salts, in which case it is not necessary to test an individual udder part/teat 101, but either the milk from only one teat 101, or the milk as collected from the whole udder. Thus, it is also possible to provide the sampling device in the milk line, i.e. a hose through which milk is transported from either the milk jar of the milk claw.

The sampling device 4 may be arranged according to any suitable sampling system, such as a valve or pump that is operable during a predetermined time in the course of the milking process, or a device that separates off a certain part of the milk stream flowing through the milk tube 3, or any alternative therefor. Since the gist of the invention proper does not reside in the specifics of the sampling device, no unnecessary details will be given here.

Figure 2 diagrammatically shows some details of the analysis device 5 of the milking system 1 according to the invention. The analysis device comprises a housing 6, with a cassette 7 with a non-shown first bobbin therein, which is rotatable around a first axle 8, and with an opening 9, from which exits an analysis tape 10 carrying analysis pads 11 separated by empty spaces 12. Used analysis tape is wound on a second bobbin 13, which a tape displacer 14 rotates around a second axle 15, under the control of a control unit 16. A sample supply line is designated 18, while a sample pump 19 provides, via the sample discharge line 20, movable by an actuator 21, a sample droplet 22. An optical sensor 23 detects colour changes.

The housing 6 of the analysis device 5 is optional, though often advantageous, to protect the analysis tape 10 and its reagent(s) from ingress of dust, water and so on. The unused analysis tape 10 is wound on a first bobbin, that is provided in the cassette 7. When the tape displacer 14 pulls the analysis tape 10, the first bobbin rotates around the first axle 8, thereby releasing, via the opening 9, a new part of the analysis tape 10, with one or more unused analysis pads 11. Advantageously, the opening 9 is narrow and/or in sealing contact with the analysis tape 10, to reduce the ingress of humidity, dust and so on, into the cassette 7.

The unused analysis pad 11 thus provided is available for analysis of a sample of the milk. Thereto, a droplet 22 of milk can be delivered to the pad 11, by having the sample pump 19 pump milk, which is received from the sample supply line 17, through the sample discharge line 20. The actuator 21, which can be or comprise a telescopic cylinder, an electromotor or any other kind of actuator, is arranged to move the sample discharge line 20, or at least the part that supplies the droplet 22, towards the analysis pad 11, and retract it therefrom.

When the sample droplet 22 has been taken up by the analysis pad 11, a colour reaction will taken place, or not, depending on the presence of a specific compound in the milk of the sample. If it is present, the intensity of the colour change in the analysis pad 11 will then depend on the concentration of the compound in the sample droplet, as well as other factors such as temperature. The colour reaction can be sensed by means of a sensor 23, in this case a camera, although alternatives such as photosensor that are sensitive to specific wavelengths, and so on, may also be useful.

The control unit 16 is operably connected to the sensor 23, in order to receive either processed data, through an integrated image processor, or the raw sensor data, and process same itself. Based on the processed data, the control unit may perform an action is necessary, such as changing or determining a destiny of the milk from the animal, either from the present milking or for a subsequent milking. It is also possible to raise an alarm, such as messaging a farmer or operator, and so on.

It is to be noted that the control unit 16 is also operably connected to the tape displacer 14, the sample pump 19, and the actuator 21. Theoretically, one or more of the mentioned parts could also have a dedicated control unit. Furthermore, in advantageous embodiments, the control unit 16 is either a part of a milking system control unit (not specifically shown here), that serves to control the milking system 1 as a whole, or is operably connected thereto. However, it is not excluded that the processed data (response of the analysis pad) are only communicated to a user, who may then take action himself.

It is also to be noted that the set-up of the analysis device 5 is not limited to the one shown here. For example, other exemplary embodiments of the analysis device 5 could comprise a set-up in which a sample droplet 22 is provided from above, wherein the sensor is provided either also above the analysis tape 10, thus observing reflected light, or from below.

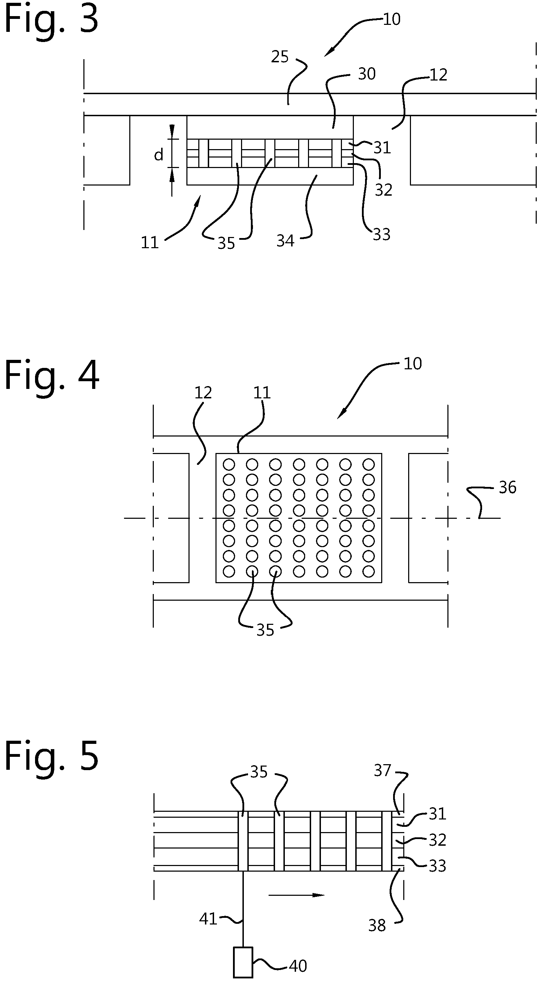

Figure 3 diagrammatically shows a cross-sectional view through an analysis tape 10 according to the invention, detailing a single analysis pad 11 on a carrier layer 25. The gap or empty space is again denoted with reference numeral 12. The analysis pad 11 here comprises a reagent pad 30, a priming pad 34, and a bonding layer therebetween, that itself comprises a first adhesive layer 31, a support layer 32, and a second adhesive layer 33. A number of holes have been designated reference numeral 35.

The analysis pad 11 is suitable to detect the presence, and/or concentration, of specific substances. In the present case, it has a reagent pad 30, that contains the reagent proper for the detection reaction, while the priming pad 34 is provided to "prime" that reaction, by changing the properties of the reaction environment. An example is for detecting milk urea nitrogen, or the urea in milk. Thereto, often reagents are used that react only, or much better, in a low pH environment. Thus, the priming pad will contain a priming agent, such as a (dry) acid, e.g. phthalic acid. After supply of the droplet 22 of the milk sample, the fluid will spread through the priming pad 34 and to the reagent pad 30, taking the priming agent with it. Of course, other types of priming are possible, such as providing a high pH environment, or an oxidising environment, and so on. The correspondingly suitable priming agent will then be provided in the priming pad 34.

It is to be noted that separating the priming agent in the priming pad 34 on the one hand, and the reagent in the reagent pad on the other depends on whether or not the priming agent would react with the reagent when in dry form. In many cases they will react, not in the least because keeping the analysis pads (absolutely) moisture-free is hardly possible. Furthermore, its use in milking machines, especially robotic milking machines, requires good reliability without human supervision during relatively long periods of time. Thus, the reagent and the priming agent must be reliably separated. Of course, the priming agent must still be able to perform its function of priming the reagent pad. Thereto, the priming pad 34 and the reagent pad 30 are intimately and reliably bonded by means of the bonding layer 31-33. The bonding layer has holes that allow fluid to pass, while keeping the priming agent and the reagent (pad) sufficiently apart in a dry condition. It is remarked that in principle the priming pad and the reagent pad could be provided the other way around, i.e. the reagent pad on top, and the priming pad 34 between the reagent pad 12 and the carrier layer 25.

The thickness d of the bonding layer is for example about 0.025 mm (1 mill) µm, although values between about 1/50th and 1/8th of a mm have also shown satisfactory properties of the analysis tape 10. The gap 12 between the analysis pads 11 serves to prevent cross-over between neighbouring analysis pads 11. They may be made by any known means, such as by providing the analysis pads 11 separately onto the carrier layer 25, or by providing the various layers 30 through 35 as continuous layers onto the carrier layer, and then cutting the gaps 12 into the required layers, such as with a laser. For this, as well as for a discussion with respect to the holes 35 in the bonding layer, reference is now made to Figure 4.

Figure 4 diagrammatically shows a top view of an analysis tape 10 according to the present invention.

The analysis tape 10 comprises the carrier layer 25 and a number of analysis pads 11, with gaps 12 therebetween. Also shown are the holes 35 that are present in the bonding layer, for ease of reference shown here as if the topmost layer, the priming layer 34, were transparent. A longitudinal line of symmetry is designated with reference numeral 36.

The holes 35 have been provided in a regular pattern. This increases the reliability of an even colour reaction over the area of the analysis pad 11. For the same reason, the pattern is symmetrical with respect to the line 36. It is noted that the larger the number of holes in a direction perpendicular to the line 36, the less important is the (exact) mirror symmetry with respect to said line. It is also noted that the square pattern shown here is not the only possible one. For example, rotating the pattern over 45 degree around an axis perpendicular to the pad 11 also provides a mirror symmetrical pattern. An advantage of this pattern is that when a laser is used to provide the hole pattern, the distance between neighbouring holes during laser burning of the holes (where the laser beam will move in a direction perpendicular to the longitudinal axis, followed by a displacement in longitudinal direction of the material) is larger than when the square pattern would be used. The larger distance between consecutively made holes means that local heating from a burned hole will influence the next hole less, leading to les risk of damaging the material around the holes. It is also possible to provide a regular pattern with a lozenge-shaped unit cell. In such a case, the long axis is preferably parallel to, or perpendicular to, the line 36. It must be borne in mind that there may then arise a slight difference in colouring in the two axial directions.

The diameter of the holes 35 in this bonding layer is e.g. 0.2 or 0.25 mm, and their hole centre to hole centre distance is between 0.4 and 0.6 mm. This provides a good compromise between strength of the layers, separation of the reagent and priming agent in a dry state, and flow rate after supply of the (milk) sample.

Fig. 5 shows a possible first step in a method of producing of the analysis tape according to the invention.

At first there is provided a bonding layer comprising the layers 30-34 as shown in Figure 3. In addition there is provided a first peel-off layer 37 and a second peel-off layer 38. A laser 40 emits a laser beam 41.

The bonding layer comprises the first adhesive layer 31, the support layer 32, and the second adhesive layer 33. To prevent premature sticking to the bonding layer, it is provided with first and second peel-off layers 37, 38. In this five-fold set of layers, the holes 35 are provided, in the case shown by shooting a laser beam 41 from a laser 40, and moving the bonding layer in the direction of the arrow. Of course, it will also be possible to direct the laser beam 41 by means of a controllable set of mirrors, so that the bonding layer need not be moved for just about every single hole 35. It is noted that it is not excluded to provide the holes 35 in a different way, such as by needle-punching or the like. However, the versatility, cleanliness and speed of a laser-based method step is preferred.

It is to be noted that in practice it would happen that the peel-off layers 37 and/or 38 would become partly stuck to the adhesive layers 31, 33, respectively, because of welding together by the laser beam. Then, peeling off the peel-off layer 37 or 38 would exert a relatively large force on the bonding layer as a whole. This could have distorted and warped the bonding layer, and in particular the holes therein, had it not been for the support layer 32, that provides strength and stability to the bonding layer as a whole. Thereto, the support layer is for example made of a polyester film.

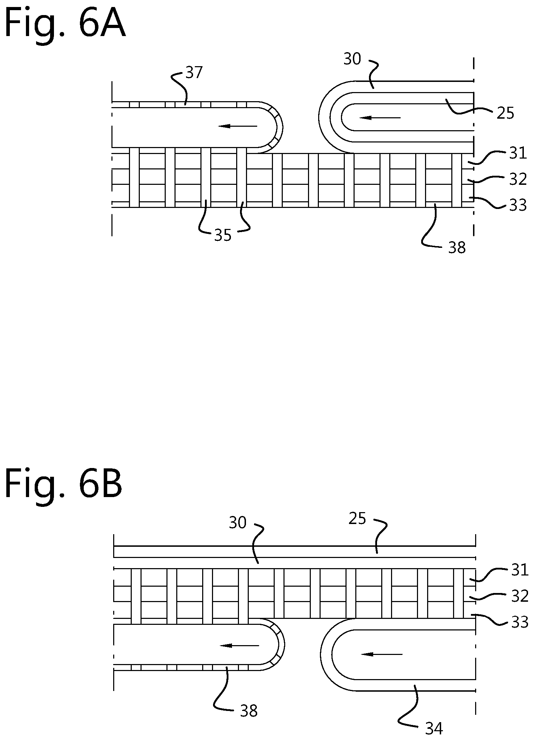

Figures 6A and 6B show further exemplary steps in a method to produce the analysis tape according to the invention.

In the step shown in Figure 6A, the first peel-off layer 37 is removed, after which the reagent pad layer 30 and the carrier layer 25 are bonded to the first adhesive layer 31, by moving same in the direction of the respective arrows. In the step shown in Figure 6B, the second peel-off layer 38 is removed, after which the priming pad layer 34 is bonded to the second adhesive layer 33.

Herein, it is to be noted that the steps of Figures 6A and 6B can be performed independently, i.e. in any order and even simultaneously. Furthermore, the carrier layer 25 and the reagent pad layer 30 may also mutually have been bonded by an additional adhesive layer (not shown here). It is also to be noted that the analysis tape shown in Figures 5 and 6A and B may later on be processed further by having a laser or other device divide the layers 30 through 34 up into separate analysis pads such as the analysis pads 11 shown in Figure 3. It is stressed here that none of the figures show any layers or pads to scale.

## Claims

1. Milking system (1) for sampling and analysing milk, comprising a milking device for obtaining milk from a dairy animal, a sampling device (4) to extract a sample from the milked milk, and an analysis device (5) for receiving and analysing the sample, wherein the analysis device comprises:
- a cassette (7) with an analysis tape (10) having a carrier layer (25) with a plurality of separate vertical flow analysis pads (11) provided thereon,
- a tape displacer (14) arranged to displace the analysis tape,
- a sample provision device arranged to provide a droplet (22) of the sample to a selected one of the analysis pads,
- a sensor (23) to sense a response in the analysis pad after provision of the sample to the selected analysis pad,
- a control unit (16) arranged to control the tape displacer, the sample provision device and the sensor, wherein each of the plurality of analysis pads (11) comprises
- a reagent pad (30), comprising a liquid-permeable material and a reagent that is arranged to react with a predetermined compound in the sample to give a response that is detectable by the analysis system,
- a priming pad (34), comprising a liquid-permeable material and a priming agent that is arranged to change a property of the sample that enhances the response, and
- a bonding layer between the reagent pad and the priming pad, the bonding layer comprising a first adhesive layer (31), a second adhesive layer (33), and a support laye (32) between said two adhesive layers, wherein the control unit (16) is arranged to process said response, and to provide an indication of the presence or concentration of said compound in said sample, wherein the bonding layer has a plurality of throughgoing holes (35), each hole having a diameter of at least about 0.15 mm.

2. Milking system according to claim 1, wherein said plurality of holes is arranged in a regular pattern, in particular a square pattern.

3. Milking system according to claim 2, wherein said pattern has a hole centre to hole centre spacing of between 1.5 times and 2.5 times said diameter of the holes.

4. Milking system according to claim 2 or 3, wherein the carrier tape has a longitudinal direction, and wherein said pattern extends mirror symmetrically with respect to said longitudinal direction.

5. Milking system according to any preceding claim, wherein the analysis pad has a maximum dimension, in particular a length, and wherein the diameter of the holes is at most 1/10th of said maximum dimension, more in particular at most 1/20th of said maximum dimension.

6. Milking system according to any preceding claim, wherein the bonding layer has a thickness in the range of 20-125 µm, preferably a thickness of 40 ± 20 µm.

7. Milking system according to any preceding claim, wherein said compound is milk urea nitrogen.

8. Milking system according to any preceding claim, wherein said priming agent comprises an acid, preferably providing a pH ≤ 2.

9. Vertical flow analysis tape (10) for milk analysis, arranged to be provided in the milking system according to any preceding claim, the analysis tape comprising a carrier layer (25) with a plurality of separate vertical flow analysis pads provided thereon, wherein each analysis pad (11) comprises
- a reagent pad (30), comprising a liquid-permeable material and a reagent that is arranged to react with a predetermined compound in the sample to give a response that is detectable by the analysis system,
- a priming pad (34), comprising a liquid-permeable material and a priming agent that is arranged to change a property of the sample that enhances the response,
**characterized in that** each analysis pad (11) further comprises:
- a bonding layer between the reagent pad and the priming pad, the bonding layer comprising a first adhesive layer (31), a second adhesive layer (33), and a support layer (32) between said two adhesive layers, wherein the bonding layer has a plurality of throughgoing holes (35), each hole having a diameter of at least about 0.15 mm.

10. Method for producing the vertical flow analysis tape (10) of claim 9, comprising the steps of
- providing a bonding layer that comprises a first adhesive layer (31), a second adhesive layer (33), and a support layer (32) between said two adhesive layers,
- making a pattern of throughgoing holes (35) in the bonding layer, preferably by means of a laser tool (40), wherein the holes have a diameter of at least about 0,15 mm,
- providing a layer of reagent pad (30) material, comprising a liquid-permeable material and a reagent that is arranged to react with a predetermined compound when supplied to the reagent pad material to give a response that is detectable by an analysis system, and providing a layer of priming pad (34) material, comprising a liquid-permeable material and a priming agent that is arranged to change a property of the sample that enhances the response, wherein one of the reagent pad material and the priming pad material is supplied on a carrier layer (25),
- bonding the bonding layer to the reagent pad material and to the priming pad material, and
- separating the bonded reagent pad material, bonding layer and priming pad material into separate analysis pads, preferably by means of laser cutting.

## Patentansprüche

1. Melksystem (1) zur Probenahme und Analyse von Milch, umfassend eine Melkvorrichtung zur Gewinnung von Milch von einem Milchtier, eine Probenahmevorrichtung (4) zur Entnahme einer Probe aus der gemolkenen Milch und eine Analysevorrichtung (5) zur Aufnahme und Analyse der Probe,
wobei die Analysevorrichtung umfasst:
- eine Kassette (7) mit einem Analyseband (10) mit einer Trägerschicht (25) mit einer Vielzahl von getrennten Vertikalfluss-Analysekissen (11), die darauf bereitgestellt sind,
- einen Bandverschieber (14), angeordnet zum Verschieben des Analysebandes,
- eine Probenbereitstellungsvorrichtung, dazu ausgelegt, einem ausgewählten der Analysekissen ein Tröpfchen (22) der Probe zuzuführen,
- einen Sensor (23) zum Erfassen einer Reaktion in dem Analysekissen nach dem Zuführen der Probe zu dem ausgewählten Analysekissen,
- eine Steuereinheit (16), angeordnet zum Steuern des Bandverschiebers, der Probenbereitstellungsvorrichtung und des Sensors,
wobei jedes der Vielzahl von Analysekissen (11) umfasst
- ein Reagenskissen (30) umfassend ein flüssigkeitsdurchlässiges Material und ein Reagens, das angeordnet ist zum Reagieren mit einer vorbestimmten Verbindung in der Probe, um eine Reaktion zu ergeben, die durch das Analysesystem nachweisbar ist,
- ein Vorbereitungskissen (34) umfassend ein flüssigkeitsdurchlässiges Material und ein Vorbereitungsmittel, angeordnet zum Verändern einer Eigenschaft der Probe, die die Reaktion verstärkt, und
- eine Haftschicht zwischen dem Reagenskissen und dem Vorbereitungskissen, wobei die Haftschicht eine erste Klebstoffschicht (31), eine zweite Klebstoffschicht (33) und eine Trägerschicht (32) zwischen den beiden Klebstoffschichten umfasst,
wobei die Steuereinheit (16) dazu ausgelegt ist, die Reaktion zu verarbeiten und eine Angabe über das Vorhandensein oder die Konzentration der Verbindung in der Probe bereitzustellen,
wobei die Haftschicht eine Vielzahl von durchgehenden Löchern (35) aufweist, wobei jedes Loch einen Durchmesser von wenigstens etwa 0,15 mm aufweist.

2. Melksystem nach Anspruch 1, wobei die Vielzahl von Löchern in einem regelmäßigen Muster, insbesondere einem quadratischen Muster, angeordnet ist.

3. Melksystem nach Anspruch 2, wobei das Muster einen Lochmitte-zu-Lochmitte-Abstand zwischen dem 1,5-fachen und dem 2,5-fachen des Durchmessers der Löcher aufweist.

4. Melksystem nach Anspruch 2 oder 3, wobei das Trägerband eine Längsrichtung aufweist und wobei das Muster spiegelsymmetrisch bezogen auf die Längsrichtung verläuft.

5. Melksystem nach einem der vorstehenden Ansprüche, wobei das Analysekissen eine maximale Abmessung, insbesondere eine Länge, aufweist und wobei der Durchmesser der Löcher höchstens 1/10 der maximalen Abmessung, insbesondere höchstens 1/20 der maximalen Abmessung, beträgt.

6. Melksystem nach einem der vorstehenden Ansprüche, wobei die Haftschicht eine Dicke in dem Bereich von 20-125 µm, vorzugsweise eine Dicke von 40 ± 20 µm, aufweist.

7. Melksystem nach einem der vorstehenden Ansprüche, wobei die Verbindung Milchharnstoff-Stickstoff ist.

8. Melksystem nach einem der vorstehenden Ansprüche, wobei das Vorbereitungsmittel eine Säure umfasst, die vorzugsweise einen pH-Wert ≤ 2 bereitstellt.

9. Vertikalfluss-Analyseband (10) für die Milchanalyse, angeordnet zur Bereitstellung in dem Melksystem nach einem der vorstehenden Ansprüche, wobei das Analyseband eine Trägerschicht (25) mit einer Vielzahl von getrennten Vertikalfluss-Analysekissen umfasst, die darauf bereitgestellt sind, wobei jedes Analysekissen (11) umfasst
- ein Reagenskissen (30) umfassend ein flüssigkeitsdurchlässiges Material und ein Reagens, das angeordnet ist zum Reagieren mit einer vorbestimmten Verbindung in der Probe, um eine Reaktion zu ergeben, die durch das Analysesystem nachweisbar ist,
- ein Vorbereitungskissen (34) umfassend ein flüssigkeitsdurchlässiges Material und ein Vorbereitungsmittel, angeordnet zum Verändern einer Eigenschaft der Probe, die die Reaktion verstärkt,
**dadurch gekennzeichnet, dass** jedes Analysekissen (11) ferner umfasst:
- eine Haftschicht zwischen dem Reagenskissen und dem Vorbereitungskissen, wobei die Haftschicht eine erste Klebstoffschicht (31), eine zweite Klebstoffschicht (33) und eine Trägerschicht (32) zwischen den beiden Klebstoffschichten umfasst,
wobei die Haftschicht eine Vielzahl von durchgehenden Löchern (35) aufweist, wobei jedes Loch einen Durchmesser von wenigstens etwa 0,15 mm aufweist.

10. Verfahren zur Herstellung des Vertikalfluss-Analysebandes (10) nach Anspruch 9, umfassend die Schritte von
- Bereitstellen einer Haftschicht, die eine erste Klebstoffschicht (31), eine zweite Klebstoffschicht (33) und eine Trägerschicht (32) zwischen den beiden Klebstoffschichten umfasst,
- Herstellen eines Musters von durchgehenden Löchern (35) in der Haftschicht, vorzugsweise mithilfe eines Laserwerkzeugs (40), wobei die Löcher einen Durchmesser von wenigstens etwa 0,15 mm aufweisen,
- Bereitstellen einer Schicht aus Reagenskissen(30)-Material, umfassend ein flüssigkeitsdurchlässiges Material und ein Reagens, das angeordnet ist, um mit einer vorbestimmten Verbindung zu reagieren, wenn es dem Reagenskissen-Material zugeführt wird, um eine Reaktion zu ergeben, die durch ein Analysesystem nachweisbar ist, und Bereitstellen einer Schicht aus Vorbereitungskissen(34)-Material umfassend ein flüssigkeitsdurchlässiges Material und ein Vorbereitungsmittel, angeordnet zum Verändern einer Eigenschaft der Probe, die die Reaktion verstärkt, wobei eines von dem Reagenskissen-Material und dem Vorbereitungskissen-Material auf einer Trägerschicht (25) bereitgestellt wird,
- Anhaften der Haftchicht an das Reagenskissen-Material und an das Vorbereitungskissen-Material, und
- Trennen des angehafteten Reagenskissen-Materials, der Haftschicht und des Vorbereitungskissen-Materials in getrennte Analysekissen, vorzugsweise mithilfe von Laserschneiden.

## Revendications

1. Système de traite (1) pour prélever et analyser du lait, comprenant un dispositif de traite pour prélever du lait d'un animal laitier, un dispositif de prélèvement (4) pour extraire un échantillon du lait laitier, et un dispositif d'analyse (5) pour recevoir et analyser l'échantillon,
le dispositif d'analyse comprenant :
- une cassette (7) avec une bande d'analyse (10) présentant une couche de support (25) sur laquelle est disposée une pluralité de tampons d'analyse d'écoulements verticaux séparés (11),
- un dispositif de déplacement de bande (14) agencé pour déplacer la bande d'analyse,
- un dispositif de fourniture d'échantillon agencé pour fournir une gouttelette (22) de l'échantillon à l'un sélectionné des tampons d'analyse,
- un capteur (23) pour détecter une réponse dans le tampon d'analyse après fourniture de l'échantillon au tampon d'analyse sélectionné,
- une unité de commande (16) agencée pour commander le dispositif de déplacement de bande, le dispositif de fourniture d'échantillon et le capteur,
chacun de la pluralité de tampons d'analyse (11) comprenant
- un tampon de réactif (30), comprenant un matériau perméable au liquide et un réactif agencé pour réagir avec un composé prédéterminé dans l'échantillon pour donner une réponse détectable par le système d'analyse,
- un tampon d'amorçage (34), comprenant un matériau perméable au liquide et un agent d'amorçage agencé pour changer une propriété de l'échantillon améliorant la réponse, et
- une couche de liaison entre le tampon de réactif et le tampon d'amorçage, la couche de liaison comprenant une première couche adhésive (31), une deuxième couche adhésive (33), et une couche de support (32) entre lesdites deux couches adhésives,
l'unité de commande (16) étant agencée pour traiter ladite réponse, et pour fournir une indication de la présence ou de la concentration dudit composé dans ledit échantillon,
la couche de liaison ayant une pluralité de trous traversants (35), chaque trou ayant un diamètre d'au moins environ 0,15 mm.

2. Système de traite selon la revendication 1, ladite pluralité de trous étant agencée selon un motif régulier, en particulier un motif carré.

3. Système de traite selon la revendication 2, ledit motif ayant un espacement entre le centre de trou et le centre de trou compris entre 1,5 fois et 2,5 fois ledit diamètre des trous.

4. Système de traite selon la revendication 2 ou 3, la bande de support ayant une direction longitudinale, et ledit motif s'étendant symétriquement par rapport à ladite direction longitudinale.

5. Système de traite selon l'une quelconque des revendications précédentes, le tampon d'analyse ayant une dimension maximale, en particulier une longueur, et le diamètre des trous étant au plus égal à 1/10 de ladite dimension maximale, en particulier au plus égal à 1/20 de ladite dimension maximale.

6. Système de traite selon l'une quelconque des revendications précédentes, la couche de liaison ayant une épaisseur dans la plage de 20-125 µm, de préférence une épaisseur de 40 ± 20 µm.

7. Système de traite selon l'une quelconque des revendications précédentes, ledit composé étant l'azote uréique du lait.

8. Système de traite selon l'une quelconque des revendications précédentes, ledit agent d'amorçage comprenant un acide, fournissant de préférence un pH ≤ 2.

9. Bande d'analyse d'écoulement vertical (10) pour l'analyse du lait, agencé pour être prévu dans le système de traite selon l'une quelconque des revendications précédentes, la bande d'analyse comprenant une couche de support (25) avec une pluralité de tampons d'analyse d'écoulements verticaux séparés prévus sur celle-ci, chaque tampon d'analyse (11) comprenant
- un tampon de réactif (30), comprenant un matériau perméable au liquide et un réactif agencé pour réagir avec un composé prédéterminé dans l'échantillon pour donner une réponse détectable par le système d'analyse,
- un tampon d'amorçage (34), comprenant un matériau perméable au liquide et un agent d'amorçage agencé pour changer une propriété de l'échantillon améliorant la réponse,
**caractérisé en ce que** chaque tampon d'analyse (11) comporte en outre :
- une couche de liaison entre le tampon de réactif et le tampon d'amorçage, la couche de liaison comprenant une première couche adhésive (31), une deuxième couche adhésive (33), et une couche support (32) entre lesdites deux couches adhésives,
la couche de liaison ayant une pluralité de trous traversants (35), chaque trou ayant un diamètre d'au moins environ 0,15 mm.

10. Procédé de production de la bande d'analyse d'écoulement vertical (10) selon la revendication 9, comprenant les étapes de
- la fourniture d'une couche de liaison qui comprend une première couche adhésive (31), une deuxième couche adhésive (33), et une couche support (32) entre lesdites deux couches adhésives,
- la réalisation d'un motif de trous traversants (35) dans la couche de liaison, de préférence au moyen d'un outil laser (40), les trous ayant un diamètre d'au moins environ 0,15 mm,
- la fourniture d'une couche de matériau de tampon de réactif (30), comprenant un matériau perméable aux liquides et un réactif agencé pour réagir avec un composé prédéterminé lorsqu'il est fourni au matériau de tampon de réactif pour donner une réponse détectable par un système d'analyse, et la fourniture d'une couche de matériau de tampon d'amorçage (34), comprenant un matériau perméable aux liquides et un agent d'amorçage conçu pour modifier une propriété de l'échantillon qui améliore la réponse, l'un parmi le matériau de tampon de réactif et le matériau de tampon d'amorçage étant fourni sur une couche support (25),
- le collage de la couche de liaison au matériau du tampon de réactif et au matériau du tampon d'amorçage, et
- la séparation du matériau de tampon de réactif lié, de la couche de liaison et du matériau de tampon d'amorçage en tampons d'analyse séparés, de préférence au moyen d'une découpe laser.
